# FASCICULE DE BREVET EUROPEEN

(11) **EP 1 874 291 B1**
(45) Date de publication et mention de la délivrance du brevet: **06.08.2008**
(21) Numéro de dépôt: 06743645.1
(22) Date de dépôt: 06.04.2006
(51) Int. Cl.: A61K 31/315, A61K 33/30, A61K 9/14, A61K 9/20, A61P 17/00

(54) **UTILISATION DU GLUCONATE DE ZINC POUR LE TRAITEMENT DE L'HIDRADENITE SUPPUREE**
VERWENDUNG VON ZINKGLUCONAT ZUR BEHANDLUNG VON HYDRADENITIS SUPPURATIVA
USE OF ZINC GLUCONATE FOR TREATING HYDRADENITIS SUPPURATIVA

(30) Priorité: 13.04.2005 FR 0503695
(43) Date de publication de la demande: 09.01.2008
(73) Titulaire: LABCATAL, 92541 Montrouge (FR)
(72) Inventeur: SUCK, Catherine, F-92330 Sceaux (FR); DRENO, Brigitte, F-44000 Nantes (FR); BODIN, Jacques, F-92300 Levallois-Perret (FR)
(74) Mandataire: L'Helgoualch, Jean
(86) Numéro de dépôt international: PCT/FR2006/000761
(87) Numéro de publication internationale: WO 2006/108949

(56) Documents cités:
- US-A- 5 208 031
- US-A1- 2005 048 139
- US-B1- 6 482 839

## Description

La présente invention concerne une nouvelle application du gluconate de zinc en thérapeutique, et plus particulièrement une nouvelle application du gluconate de zinc administré par voie orale pour le traitement de l'hidradénite suppurée communément dénommée maladie de Verneuil.

La maladie de Verneuil, ou hidradénite suppurée, est une maladie orpheline, c'est-à-dire une maladie relativement peu répandue, pour laquelle peu de traitement efficaces ont été proposés.

La maladie de Verneuil est une affection chronique évoluant par poussées inflammatoires et suppuratives des zones anatomiques comportant des glandes sudorales apocrines. Les structures anatomiques qui sont atteintes sont celles de l'unité pilo-sébacée et sudorale (les glandes sudorales apocrines s'abouchant dans le follicule pileux). La lésion initiale est probablement une obstruction du follicule pileux, les glandes sudorales apocrines étant atteintes secondairement. Cliniquement, la maladie est caractérisée au début par l'apparition périodique de nodules douloureux profonds sans ouverture spontanée à la peau, à la différence de l'acné et des furoncles. La répétition des poussées de nodules inflammatoires et leur ouverture secondaire à la peau entraînent la formation de fistules suppuratives douloureuses et malodorantes, le processus cicatriciel formant des brides qui peuvent aboutir à la formation de vastes placards infiltrés suppuratifs et douloureux.

Le diagnostic repose sur trois éléments : des lésions élémentaires caractéristiques, une topographie spécifique et le caractère chronique ou récidivant. Les lésions élémentaires caractéristiques sont les nodules hypodermiques fermes, douloureux ou sensibles, de plus d'un centimètre de diamètre sans ouverture spontanée à la peau (du moins au début), les abcès, les fistules, les placards infiltrés, les brides cicatricielles et les comédons polypores. Les lésions inflammatoires ont chacune une durée moyenne de 7 jours, mais la répétition des poussées et la coexistence des éléments fait que plus de 50 % des patients ont en permanence un ou plusieurs nodules douloureux. Les topographies caractéristiques sont les aisselles, les régions sous et inter-mammaires, les régions inguino-crurales et péri-anales. On observe plus rarement une atteinte des fesses, du pubis, de la poitrine et des zones rétro-auriculaire. La durée d'évolution moyenne de la maladie est de 19 ans, le début survenant fréquemment autour de la puberté ou chez l'adulte jeune et la maladie ayant tendance à disparaître au-delà de 50 ans.

La gravité est extrêmement variée, depuis quelques nodules par an jusqu'à la permanence d'un énorme placard suppuré. Une classification de cette sévérité a été établie par Hurley en 3 stades. Elle permet de classer les malades entre ceux qui relèvent probablement d'un traitement médical au stade I et ceux qui relèvent à coup sûr d'une exérèse chirurgicale large au stade III. Les complications de l'hidradénite suppurée sont rares, mais certaines sont graves tels les rhumatismes inflammatoires axiaux ou périphériques. La fréquence globale des cancers est augmentée chez ces sujets : odd ratio (OR) = 1,5 ; cancers hépatiques et buccaux mais surtout cancers épithéliaux, certains survenant essentiellement dans les localisations génito-crurales et anales avec un odd ratio à 4,6.

Diverses études ont tenté d'apprécier la prévalence qui est estimée à 1 % de la population globale, et peut monter jusqu'à 4 % dans les populations d'adultes jeunes.

Aucun traitement n'est parfaitement satisfaisant et cela explique le désarroi des patients atteints de cette affection souvent très invalidante. Même dans les formes "mineures" la répétition des poussées douloureuses et de suppuration parfois malodorante entraîne une répercussion sévère sur la qualité de vie.

Au stade III et souvent au stade II de Hurley, le seul recours est l'exérèse chirurgicale de toute la zone atteinte suivie d'un recouvrement qui se fait souvent en deuxième intention avec ou sans greffe.

Au stade I et I-II, divers traitements médicaux ont été essayés avec des résultats mitigés. Les anti-androgènes comme l'acétate de cyprotérone n'ont montré une efficacité qu'à des doses importantes (100 mg/jour d'acétate de cyprotérone) chez un faible nombre de patients avec une récidive lors du passage à 50 mg.

La radiothérapie a permis d'obtenir une rémission chez certains patients, sans effet secondaire observé. Cependant compte tenu du risque spécifique spontané de cancer des sujets atteints d'hidradénite suppurée, l'utilisation de radiothérapie comporte un risque potentiel à long terme qui paraît déraisonnable.

Des essais ont été faits récemment avec les lasers épilatoires avec des résultats encourageants.

L'isotrétinoïne per os a donné des résultats très médiocres dans une étude ouverte de 80 patients. Ce résultat n'est pas surprenant compte tenu de l'absence d'hypersécrétion sébacée dans cette maladie contrairement à l'acné.

Les antibiotiques ont été largement utilisés parfois dans l'optique d'une maladie d'origine primitivement infectieuse, hypothèse aujourd'hui caduque : la flore est habituellement poly-microbienne comportant des staphylocoques, des streptocoques, des Bacilles à gram négatif et des anaérobies. Les traitements courts, soit par anti-staphylococciques, soit par clindamycine, donnés au début d'une poussée dans l'espoir de l'arrêter donnent des résultats assez médiocres. Les essais de traitements au long cours par les cyclines, sur le modèle de ce qui est fait dans l'acné, dans le but de prévenir l'apparition des poussées n'ont pas procuré de résultat satisfaisant.

Il n'y a donc aujourd'hui aucun traitement médical satisfaisant pour atténuer ou supprimer les poussées inflammatoires de la maladie dans ses stades précoces.

On sait que certains sels de zinc peuvent être utilisés en thérapeutique pour le traitement de certaines affections dermatologiques. Plus particulièrement des spécialités à base de gluconate de zinc sont utilisées par voie orale pour le traitement de l'acné inflammatoire macrokystique ou nodulaire, ou de l'acrodermatite entéropathique (voir dictionnaire Vidal, Editions OVP, Paris 2004). Dans le traitement de l'acné, le gluconate de zinc peut aussi être associé au glycolate de chitosan selon le brevet EP 981.325 et à un extrait végétal riche en tannins selon le brevet FR 2.765.108. On a également proposé d'utiliser le gluconate de zinc pour le traitement de la sclérose en plaque comme dans le brevet EP 184.514 ou pour le traitement des hyperprolactinémies comme dans le brevet EP 185.586. Le brevet US 5.208.031 décrit l'utilisation de l'acétate, du propionate et du gluconate de zinc pour le traitement de l'herpès. Plus récemment, la demande de brevet US 2005/048139 a proposé des compositions associant des sels de zinc tels que le gluconate, le lactate et l'acétate de zinc pour diminuer l'effet anti-irritant de composés antimicrobiens utilisés dans des compositions hygiéniques.

L'hidradénite suppurée et l'acné sont deux maladies différentes. Jusqu'ici les traitements de l'acné n'ont entraîné dans l'hidradénite suppurée que des résultats médiocres et/ou transitoires.

Les études et expérimentations effectuées par la demanderesse ont révélé de manière inattendue une bonne efficacité anti-inflammatoire pour des doses quotidiennes supérieures aux doses utilisées habituellement dans le traitement de l'acné, et ont aussi permis de démontrer que le gluconate de zinc pouvait être utilisé efficacement dans le traitement de la maladie de Verneuil.

Ainsi la présente invention a pour objet l'utilisation du gluconate de zinc dans la préparation d'un médicament destiné au traitement de l'hidradénite suppurée, et plus particulièrement un médicament administré par voie orale.

L'invention a également pour objet l'utilisation d'une nouvelle composition à base de gluconate de zinc utilisable en thérapeutique, présentée sous une forme administrable par voie orale, destinée au traitement de l'hidradénite suppurée, et spécialement formulée pour ce traitement.

Selon la présente invention, la dose unitaire, c'est-à-dire la teneur en gluconate de zinc dans la composition, est généralement comprise entre 15 et 90 mg par unité de prise, et de préférence entre 15 et 45 mg.

La posologie journalière est déterminée par le praticien en fonction de l'état du patient, mais elle est généralement comprise entre 45 et 125 mg.

La tolérance à ces doses (par exemple 90 mg/jour, soit 6 gélules dosées à 15 mg) est le plus souvent bonne, les effets secondaires observés sont seulement digestifs, et il n'y a pas de toxicité systémique à redouter (la monographie du "Vidal", à la rubrique "surdosage", est ainsi rédigée : "La probabilité d'une intoxication aiguë est nulle...")

L'efficacité du gluconate de zinc dans le traitement de l'hidradénite suppurée a été vérifiée par l'observation clinique effectuée sur 20 patients dont 16 ont été suivis pendant une période pouvant aller jusqu'à 24 mois.

20 patients, 14 femmes et 6 hommes, âgés de 23 à 72 ans, dont l'ancienneté de la maladie varie de 6 à 13 ans, ont été mis sous 90 mg de gluconate de zinc par jour pendant une période de quelques mois à 3 ans.

Dans 34% des cas la localisation des lésions était inguinale, axillaire dans 24% des cas, fessière dans 17% des cas. Les autres régions touchées dans 5% à 7% des cas étaient la poitrine, le pubis ou le périnée.

Suivis pendant plusieurs, mois ces patients voient leur maladie en rémission complète pour 9 (45%) d'entre eux, en rémission partielle pour 6 (30%) d'entre eux. 4 patients ne sont pas renseignés (traitement instauré trop récemment) et pour un patient seulement le traitement s'avère inefficace.

Ces données sont reproduites dans le tableau ci- après :

| N | Sexe | Localisation | Dose 1 | Dose 2 | Rep | suivi |
|---|---|---|---|---|---|---|
| 1 | F | plis inguinaux, fesses, creux axillaires | 90 mg | 60 mg | RC | 3 mois |
| 2 | M | ND | 75 mg | 75 mg | RC | > 12 mois |
| 3 | M | axillaires, présternale, inguinales | 90 mg | 60 mg | RP | 8 mois |
| 4 | M | bourses, inguinales, fesses | 90 mg | | RP | 7 mois |
| 5 | F | axillaires, périmammaire | 90 mg | 90 mg | RP | > 12 mois |
| 6 | F | inguinales, sus pubien, seins, pli inter fessier | 90 mg | | RP | 6 mois |
| 7 | M | axillaires, pelviennes | 90 mg | 30 mg | RC | > 12 mois |
| 8 | F | axillaires, inguinales | 90 mg | | | |
| 9 | F | axillaires | 90 mg | 15 mg | RC | > 12 mois |
| 10 | F | inguinales, interfessier, péri-vulvaire | 120 mg | ND | RP | ND |
| 11 | F | axillaires, inguinales | 90 mg | | ineff. | |
| 12 | F | inguinales, fesses, axillaires | 90 mg | 60 mg | RC | 4 mois |
| 13 | F | axillaires, inguinales | 90 mg | | | |
| 14 | M | inguinales, fesses, abdomen | 90 mg | 15 mg | RC | 4 mois |
| 15 | M | axillaires, inguinales | 120 mg | | RP | 5 mois |
| 16 | F | ND | 60 mg | | | |
| 17 | F | inguinales, axillaires, fesses | 90 mg | 30 mg | RC | > 12 mois |
| 18 | F | inguinales | 60 mg | | RC | 7 mois |
| 19 | F | inguinales | 90 mg | 30 mg | RC | > 12 mois |
| 20 | F | inguinales | 90 mg | ND | | ND |

| | | | | | | |
|---|---|---|---|---|---|---|
| RC : rémission complète RP : rémission partielle ND : non documenté | | | | | | |

La "dose 1" est la dose administrée en début de traitement. La "dose 2" est la dose d'entretien. Le suivi médical a été généralement de 4 à 8 mois, et dans quelques cas il a été prolongé au-delà de 12 mois.

Ces résultats montrent que le gluconate de zinc présente une efficacité satisfaisante dans le traitement de l'hydradénite suppurée lorsqu'il est administré par voie orale à des doses relativement élevées, c'est-à-dire de l'ordre de 60 à 120 mg par jour pour le traitement d'attaque, et 15 à 90 mg par jour pour le traitement d'entretien.

La composition utilisée selon l'invention peut être présentée sous forme de gélules, de capsules, de comprimés, de soluté buvable, de comprimés effervescents ou de toute autre forme usuelle pour administration par voie orale.

La composition, outre le gluconate de zinc, peut contenir des excipients adaptés en fonction de l'usage envisagé, tels que des agents diluants, par exemple le lactose ou le mannitol, des agents lubrifiants, par exemple le stéarate de magnésium ou la silice colloïdale hydratée, un agent désintégrant par exemple l'amidon de blé ou l'amidon prégélatinisé.

D'autres excipients peuvent être utilisés en fonction du mode d'administration envisagé.

Des exemples de réalisation nullement limitatifs sont donnés ci-après pour illustrer l'invention.

### Formulation 1 : gélule

Une gélule ayant la composition suivante est préparée par les techniques usuelles :

| | | |
|---|---|---|
| Gluconate de zinc (exprimé en zinc métal) | | 15,0 mg |
| Stéarate de magnésium | | 6,0 mg |
| Lévilite | | 6,0 mg |
| Tixosil | | 2,0 mg |
| Amidon de blé | | 27,7 mg |
| Lactose | q.s.p. | 200,0 mg |

Ces quantités sont indiquées pour une gélule de taille 2

### Formulation 2 : comprimé

En utilisant les techniques usuelles, on prépare un comprimé ayant la composition suivante:

| | | |
|---|---|---|
| Gluconate de zinc (exprimé en zinc métal) | | 15,0 mg |
| PEG 6000 | | 5,76 mg |
| Primojel | | 7,2 mg |
| Compritol 888 | | 5,76 mg |
| Sorbitol | q.s.p. | 210,0 mg |

### Formulation 3 : comprimé pelliculé

En utilisant les techniques usuelles, on prépare un comprimé ayant la composition suivante:

| | | |
|---|---|---|
| Gluconate de zinc (exprimé en zinc métal) | | 15,0 mg |
| PEG 6000 | | 5,76 mg |
| Primojel | | 7,2 mg |
| Compritol 888 | | 5,76 mg |
| Sepisperse | | 8,2 mg |
| HP50 | | 10,81 mg |
| Phtalate de dibutyle | | 1,09 mg |
| Sorbitol | q.s.p. | 210,0 mg |

### Formulation 4 : comprimé effervescent

Un comprimé effervescent ayant la composition suivante est préparé par les techniques usuelles :

| | | |
|---|---|---|
| Gluconate de zinc (exprimé en zinc métal) | | 30,0 mg |
| Acide citrique anhydre | | 0,96 g |
| PVP K90 | | 1,0 mg |
| Saccharine sodique | | 5,0 mg |
| Bicarbonate de sodium | | 1,194g |
| Carbonate de sodium | | 60,0 m g |
| L-leucine | | 5,76 mg |
| Sepisperse | | 125,0 mg |
| HP50 | | 10,81 mg |
| Phtalate de dibutyle | | 1,09 mg |
| Mannitol | qsp un comprimé de | 2,657 g |

### Formulation 5 : gélule

Une gélule ayant la composition suivante est préparée par les techniques usuelles :

| | | |
|---|---|---|
| Gluconate de zinc (exprimé en zinc métal) | | 45,0 mg |
| Stéarate de magnésium | | 6,0 mg |
| Lévilite | | 6,0 mg |
| Tixosil | | 2,0 mg |
| Amidon de blé | | 27,7 mg |
| Lactose | q.s.p. | 300,0 mg |

### Ces quantités sont indiquées pour une gélule de taille 1

### Formulation 6 : sachet de poudre effervescente

Un sachet de poudre effervescente ayant la composition suivante est préparé par les techniques usuelles :

| | | |
|---|---|---|
| Gluconate de zinc (exprimé en zinc métal) | | 30,0 mg |
| Acide citrique anhydre | | 1,086 g |
| Aspartame | | 20,0 mg |
| Bicarbonate de sodium | | 1,316 g |
| Carbonate de sodium | | 60,0 mg |
| L-leucine | | 75,0 mg |
| Mannitol | qsp un sachet de | 3,0 g |

## Revendications

1. L'utilisation du gluconate de zinc pour la fabrication d'un médicament pour le traitement de l'hidradénite suppurée.

2. Utilisation selon la revendication 1, **caractérisée en ce que** le gluconate de zinc est sous forme adaptée à une administration par voie orale.

3. Utilisation selon la revendication 2, **caractérisée en ce que** le gluconate de zinc est présenté sous forme de comprimé, comprimé effervescent, de gélule, de capsule, de soluté buvable ou de poudre.

4. Utilisation selon la revendication 3, **caractérisée en ce que** le gluconate de zinc est présenté en doses unitaires comprises entre 15 et 90 mg.

5. Utilisation selon l'une quelconque des revendications précédentes, **caractérisée en ce que** la posologie journalière est comprise entre 45 et 125 mg.

## Claims

1. The use of zinc gluconate for the manufacture of a medicament for treating suppurative hidradenitis.

2. The use according to claim 1, **characterized in that** the zinc gluconate is in a form suitable for oral administration.

3. The use according to claim 2, **characterized in that** the zinc gluconate is in the form of a tablet, an effervescent tablet, a gel capsule, a wafer capsule, a drinkable solution or a powder.

4. The use according to claim 3, **characterized in that** the zinc gluconate is in unit doses of between 15 and 90 mg.

5. The use according to any one of the preceding claims, **characterized in that** the daily dosage is between 45 and 125 mg.

## Patentansprüche

1. Verwendung von Zinkgluconat zur Herstellung eines Medikaments zur Behandlung von Hidradenitis suppurativa.

2. Verwendung nach Anspruch 1, **dadurch gekennzeichnet, dass** das Zinkgluconat in einer Form vorliegt, die für eine Verabreichung auf oralem Weg geeignet ist.

3. Verwendung nach Anspruch 2, **dadurch gekennzeichnet, dass** das Zinkgluconat in Form einer Tablette, einer Brausetablette, einer Gelatinekapsel, einer Kapsel, einer trinkbaren Lösung oder eines Pulvers vorliegt.

4. Verwendung nach Anspruch 3, **dadurch gekennzeichnet, dass** das Zinkgluconat in Einheitsdosen zwischen 15 und 90 mg vorliegt.

5. Verwendung nach einem der vorangegangenen Ansprüche, **dadurch gekennzeichnet, dass** die Tagesdosis zwischen 45 und 125 mg liegt.
